# EUROPEAN PATENT SPECIFICATION

(11) **EP 1 246 626 B1**
(45) Date of publication and mention of the grant of the patent: **26.10.2005**
(21) Application number: 01901338.2
(22) Date of filing: 12.01.2001
(51) Int. Cl.: A61K 31/70, A61P 3/08, A61P 3/10

(54) **USE OF A CYCLIC ETHER FOR THE PREPARATION OF MEDICAMENTS AFFECTING GLUCOSE TOLERANCE**
VERWENDUNG EINES ZYKLISCHEN ETHERS ZUR HERSTELLUNG VON MITTELN, DIE DIE GLUKOSETOLERANZ BEEINFLUSSEN
UTILISATION D'UN ETHER CYCLIQUE DANS LA PREPARATION DE MEDICAMENTS AGISSANT SUR LA TOLERANCE AU GLUCOSE

(30) Priority: 14.01.2000 GB 0000888
(43) Date of publication of application: 09.10.2002
(73) Proprietor: DANISCO A/S, 1001 Copenhagen K. (DK)
(72) Inventor: AHREN, Bo, S-224 57 Lund (SE); YU, Shukun, S-212 40 Malmoe (SE)
(74) Representative: Alcock, David
(86) International application number: PCT/IB2001/000139
(87) International publication number: WO 2001/051058

(56) References cited:
- AHREN BO ET AL: "1,5-Anhydro-D-fructose increases glucose tolerance by increasing glucagon-like peptide-1 and insulin in mice." EUROPEAN JOURNAL OF PHARMACOLOGY, vol. 397, no. 1, 2000, pages 219-225, XP000999803 ISSN: 0014-2999
- KAMETANI SHUNICHI ET AL: "Hepatic production of 1,5-anhydrofructose and 1,5-anhydroglucitol in rat by the third glycogenolytic pathway." EUROPEAN JOURNAL OF BIOCHEMISTRY, vol. 242, no. 3, 1996, pages 832-838, XP000986264 ISSN: 0014-2956

## Description

### Field of the Invention

The present invention relates to the modulation of glucose tolerance disorders, such as diabetes.

### Background to the Invention

Diabetes is a major health issue, affecting over 1 million people in the United Kingdom alone. It has been estimated that diabetes and its sequelae account for as much as 5-6% of total National Health Service spending.

The development of type-2 diabetes is caused by a failure of the pancreas to secrete insulin in sufficient quantities. Type-2 diabetes is often preceded by a period of insulin resistance. Insulin resistance is caused by impairment of the ability of insulin to properly regulate glucose metabolism.

Glycogen is the main polymer of carbohydrates such as glucose in liver and muscles for carbon and energy storage and as a dynamic pool for maintaining glycemia (i.e. glucose) homeostasis. Both the synthesis and degradation of glucose are known to be under rigid control at both enzymatic and hormone levels, and numerous diseases are known to be related to disorders of glycogen metabolism, including diabetes (Larner, 1990).

The breakdown of glycogen may proceed via a number of metabolic routes. Glycogen may be broken down by α-glucosidase giving rise to free glucose molecules. Glycogen may also be degraded by glycogen phosphorylase, producing glucose-1-phosphate, which can be converted to free glucose by a phosphatase. (Larner, 1990).

Glycogen may be also be broken down by via the Anhydrofructose Pathway in which glycogen is converted to 1,5-anhydro-D-fructose ("1,5AnFru") by α-1,4-glucan lyase (Yu *et al*., 1999), which is then further metabolised. One of the products of this metabolic pathway is 1,5-anhydro-D-glucitol (1,5AnGlc-ol). 1,5AnGlc-ol is found in the cerebrospinal fluid and in plasma in humans and may be secreted into the urine. The level of 1,5AnGlc-ol is around 20-40 µg ml⁻¹ plasma in normal persons but in diabetic patients it is found at a reduced level of about 0-10 µg ml⁻¹ plasma (Yamanouchi *et al.,* 1989; Stickle and Turk, 1997).

This alternative glycogen degrading route has been demonstrated in *Escherichia coli,* fungi and algae (for review see Yu *et al*., 1999) and also in livers in rats (Kametani *et al*., 1996). 1,5AnFru occurs in free state, for example, at about 0.4 µg g⁻¹ fresh rat liver tissue and up to about 1.9 mg g⁻¹ fresh tissue of the red alga *Gracilariopsis leameiformis* (Broberg *et al.,* 1999).

Considerable effort has been directed towards understanding the molecular basis of diabetes, and to the provision of therapeutics for the alleviation of glucose intolerance.

### Summary Aspects Of The Present Invention

It has now been surprisingly shown that cyclic ethers - in particular 1,5-anhydro-D-fructose (1,5AnFru) or derivatives that are based on 1,5AnFru or derived from 1,5AnFru - are useful in the modulation of glucose metabolism in mammals, in particular in the increase of glucose tolerance.

In accordance with the present invention, we have found that glucose metabolism can be modulated using a medicament comprising a cyclic ether. Preferably, said cyclic ether is, or is derivable from or is based on, 1,5AnFru. In more detail, the cyclic ether may modulate specific proteins that are involved in glucose metabolism, such as Glucagon-Like Peptide 1 (GLP-1), or insulin.

Thus, one aspect of the present invention concerns a composition for use in or as a pharmaceutical (otherwise called a medicament), wherein said composition comprises a cyclic ether which modulates glucose metabolism. Preferably, said cyclic ether acts via proteins that are involved in glucose metabolism.

For ease of reference, these and further aspects of the present invention are now discussed under appropriate section headings. However, the teachings under each section are not necessarily limited to each particular section.

### Detailed Aspects Of The Present Invention

According to one aspect, the present invention provides a pharmaceutical for the modulation of glucose metabolism.

More particular, the present invention provides the use of a cyclic ether in the manufacture of a medicament to treat a glucose tolerance disorder and/or to modulate GLP-1.

According to another aspect, the present invention provides the use of a cyclic ether as a medicament.

Further, the present invention provides the use of a cyclic ether for the manufacture of a medicament for the modulation of glucose metabolism.

Accordingly the present invention provides the use of a cyclic ether, or a pharmaceutically acceptable form (e.g. salt) thereof, in the manufacture of a medicament to treat a glucose tolerance disorder and/or to modulate GLP-1.

The present invention also provides a composition for use in medicine, said composition comprising: a cyclic ether as an active ingredient; and optionally a pharmaceutically acceptable carrier, diluent or excipient; wherein said cyclic ether is capable of affecting glucose tolerance and/or modulating GLP-1.

The present invention also provides a process for preparing a composition according to the present invention, said process comprising the steps of: providing a cyclic ether according to the present invention; and admixing said cyclic ether with a pharmaceutically acceptable carrier, diluent or excipient.

The present invention also provides the use of 1,5AnFru in the manufacture of a medicament for affecting glucose tolerance disorder and/or modulating GLP-1.

### Preferable Aspects

Preferably the cyclic ether is a sugar.

Preferably the cyclic ether has a hexose ring.

Preferably the cyclic ether is selected from one or more of: 1,5AnFru, an active mimic of 1,5AnFru, a cyclic ether based on 1,5AnFru, a cyclic ether derived from 1,5AnFru - such as a tautomer or hydrate thereof, or the dehydration product of 1,5AnFru, or a tautomer or hydrate thereof, or derivatives thereof.

More preferably, the cyclic ether is 1,5AnFru.

Preferably, the medicament or composition is used to treat diabetes.

### Advantages

The present invention is advantageous since it provides compounds that are capable of treating a glucose tolerance disorder and/or modulating GLP-1

In accordance with a highly preferred aspect of the present invention, we present results indicating that 1,5AnFru and its isomers and derivatives can increase the glucose tolerance by increasing the incretin glucagon-like peptide 1 (GLP-1) and insulin level in blood after oral intake of 1,5AnFru.

Thus, the present invention provides compounds that can modulate glucose metabolism.

### Glycogen/Glucose Metabolism

Glycogen is the main polymer of carbohydrate in liver and muscles for carbon and energy storage and as a dynamic pool for maintaining glycemia homeostasis. Both the synthesis and degradation are known to be under rigid control at both enzymatic and hormone levels and numerous diseases are known to be related to the metabolism disorder of glycogen (Mathews and van Holde, 1990).

The breakdown of glycogen is catalysed by α-glucosidase to free glucose and by glycogen phosphorylase to glucose-1-phosphate, which can be converted to free glucose by a phosphatase. (Larner, 1990). Novel proteins, such as glycogenin (Alonso *et al.,* 1995) and PTG (protein targeting to glycogen; Printen *et al*., 1997) are also thought to be involved in these processes.

As indicated above, a further glycogen degradation route, is the Anhydrofructose Pathway (Yu *et al*., 1999). In this pathway, glycogen is first converted to 1,5-anhydro-D-fructose (1,5AnFru) by α-1,4-glucan lyase (Yu *et al.,* 1999); the formed 1,5AnFru is reduced by a NADPH-dependent 1,5AnFru specific reductase to 1,5-anhydro-D-glucitol (1,5AnGlc-ol), which may further be phosphorylated to 1,5AnGlc-ol 6-phosphate by a kinase (Shiga *et al*., 1999, Yu *et al.,* 1999).

The physiological function of this alternative glycogenolytic pathway has been elucidated in fungi, algae and *E. coli.* For example, the metabolites of this pathway regulate glucose uptake, glycogen synthesis and degradation in *E. coli* (Shiga *et al*., 1999). In contrast, however, little is known in mammals about the physiological importance of this alternative glycogen degrading route and its impact on the homeostasis of glycogen and carbon metabolism in general.

The current study examines the effect of 1,5AnFru on glucose homeostasis and the secretion of insulin in mice. The results obtained indicate that orally administered 1,5AnFru increases glucose tolerance and insulin secretion following an oral but not an intravenous glucose tolerance.

We then investigated whether 1,5AnFru affects plasma levels of glucagon-like peptide-1 (GLP-1) following oral glucose, since GLP-1 is a main gut hormone regulating islet hormone secretion (Ahrén, 1998).

### Modulation of Glucose Metabolism

The present invention is advantageous in that it provides compounds that are capable of modulating glucose metabolism.

Modulation of glucose metabolism refers to an effect on glucose metabolism such as increasing or decreasing the sequestration of glucose, the polymerisation or depolymerisation of glucose, for example into or out of glycogen or starch or long-chain sugars or other polymeric forms of glucose. Modulation of glucose metabolism may also refer to the catabolism of glucose, such as via the citric acid cycle or other routes of degradation. Modulation of glucose metabolism may also refer to the attenuation of associated signals such as via insulin, or via GLP-1, or other polypeptide in some way associated with glucose metabolism.

### Insulin

The cyclic ether of the present invention affects (directly or indirectly) insulin action.

The term "insulin action" includes the action of insulin itself or an entity capable of affecting the action of insulin.

The term "affects insulin action" is used herein to mean that insulin action is enhanced, increased, augmented, inhibited, reversed, down-regulated or in some way modulated.

The term "affects" is also intended to include mimicking of the effect(s) of insulin, altering the endogenous effect(s) of insulin, or modulating one or more of the effect(s) of insulin. These effect(s) of insulin may be those found in cells or tissues derived from an organism affected by diabetes, or glucose intolerance, or may be found in cells or tissues derived from an organism which is not affected by diabetes or glucose intolerance.

An insulin associated tissue is any tissue which is either known or suspected of being involved in some way with insulin. This involvement may be direct, such as tissues which produce insulin, or which control or affect the production of insulin. An insulin associated tissue may be one which responds to insulin in some way, for example by altering its metabolism in response to the presence or absence of insulin, or one which has developed resistance to the action of insulin, or resistance to the presence or absence of insulin.

An "insulin target tissue" is a tissue in which insulin has an effect. This term includes tissues in which insulin would normally have an effect, but which may have developed resistance to the action of insulin. This term also includes tissues in which insulin would not normally have an effect, but may have developed a sensitivity to the action of insulin. Such tissues include muscle, fat or liver.

Determining whether a cyclic ether affects or mimics insulin refers to the assessment of one or more of the effects of insulin in the presence and absence of the cyclic ether, and deciding whether the cyclic ether has influenced one or more of these characteristic(s) or effect(s). Examples of effects of insulin which might be monitored in order to determine whether or not a cyclic ether affects or mimics insulin may include measuring the expression levels of one or more molecules believed to be involved in insulin signalling or glucose metabolism. Other effects which might be monitored include, but are not limited to, measuring the stimulation of one or more of the glucose metabolism and/or insulin-related signalling pathways, monitoring the levels of glycogen synthesis or breakdown, or assessing the activity of enzymes such as glycogen synthase. If any of these characteristics or effects is found to be different in the presence or absence of one or more cyclic ethers, or if the level of insulin or GLP-1 or other such molecule(s) are altered, then said cyclic ethers would be considered to have affected or mimicked insulin action.

There exist immortalised muscle cell lines which are currently used for studies of insulin action, for example those derived from rodents (e.g. L6 and C2C12). Such prior art rodent cell lines are therefore of value in studying aspects of glucose metabolism or glucose tolerance.

### Diabetes

In a preferred aspect, the present invention is directed to the treatment of diabetes.

Numerous gene products are linked with glucose metabolism disorders such as diabetes, for example those shown below are extracted from the OMIM Morbid Map (http://www.ncbi.nlm.nih.gov/Omim/searchmorbid.html) which presents the cytogenetic map location of disease genes.

| Disorder | Symbol(s) | Location |
|---|---|---|
| Diabetes insipidus, nephrogenic | AVPR2, DIR, DI1, ADHR | Xq28 |
| Diabetes insipidus, nephrogenic, autosomal dominant | AQP2 | 12q13 |
| Diabetes insipidus, nephrogenic, autosomal recessive | AQP2 | 12q13 |
| Diabetes insipidus, neurohypophyseal | AVP, AVRP, VP | 20p13 |
| Diabetes mellitus, insulin-dependent, neonatal | PBCA | Chr.6 |
| Diabetes mellitus, insulin-resistant, with acanthosis nigricans | INSR | 19p13.2 |
| Diabetes mellitus, rare form | INS | 11p15.5 |
| Diabetes mellitus, type II | GCGR | 17q25 |
| Diabetes mellitus, type II | NEUROD1, NIDDM | 2q32 |

The medicament compositions comprising cyclic ethers described herein may act in modulating diabetes related polypeptides, such as any of those shown in the above table or such as GLP-1 or insulin (discussed in more detail herein), or such as any other polypeptide related to glucose metabolism.

### Glucagon-Like Peptide 1 (GLP-1)

In one aspect, the present invention relates to the modulation of GLP-1.

In this respect, it is known that GLP-1 is involved in the gut control of postprandial insulin secretion as an incretin hormone. It is also known that GLP-1 exerts antidiabetogenic actions caused by increased insulin secretion, reduced glucagon secretion and inhibited gastric emptying (Nauck *et al*., 1997; Holst *et al*., 1998; Nauck 1998; Ahrén 1998).

For the development of GLP-1 in the treatment of diabetes, however, exogenous GLP-1 administration has major limitations due to its short half life, being less than 1.5 min in humans (Deacon *et al*., 1995) and the need to administer the peptide parenterally due to fast gastrointestinal degradation. Attempts to circumvent these limitations include alternative routes of administration, such as buccal (Gutniak *et al*., 1997), combined inhibition of the GLP-1 degrading enzyme, dipeptidyl peptidase IV (DDP-IV; Holst and Deacon, 1998), and the use of DPPIV-resistant analogues (Deacon *et al*., 1998). The present invention overcomes some or all of these problems.

Background teachings on GLP-1 have been presented by Victor A. McKusick *et al* on http://www.ncbi.nlm.nih.gov/Omim. For ease of reference, the following information has been extracted from that source.
Glucagon-like peptide-1 (GLP1) is a hormone derived from the preproglucagon molecule (138030) and is secreted by intestinal L cells. It is the most potent stimulator of glucose-induced insulin secretion and also suppresses in vivo acid secretion by gastric glands. By transient expression of a rat pancreatic islet cDNA library in COS cells, Thorens (1992) isolated a cDNA for the GLP1 receptor (GLP1R). Transfected into COS cells, the receptor bound GLP1 with high affinity and was coupled to activation of adenylate cyclase. It did not bind peptides of related structure and similar function, such as glucagon (GCG; 138030), gastric inhibitory polypeptide (GIP; 137240), vasoactive intestinal peptide (VIP; 192320), or secretin (SCT; 182099). The receptor is 463 amino acids long and contains 7 transmembrane domains. Sequence homology was found only with the receptors for secretin (SCTR; 182098), calcitonin (CALCR; 114131), and parathyroid hormone (PTHR; 168468), which together form a newly characterized family of G-coupled receptors. Dillon *et al*. (1993) also cloned a cDNA corresponding to the GLP1R gene. Stoffel *et al*. (1993) localized the GLP1R gene to 6p21 by fluorescence in situ hybridization. Kershaw *et al*. (1995) reported the genetic mapping of mouse Glp1r centromeric to the major histocompatibility region on proximal chromosome 17.

### Cyclic Ether

The present invention relates to the use of a cyclic ether as a medicament.

The cyclic ether has the Formula III 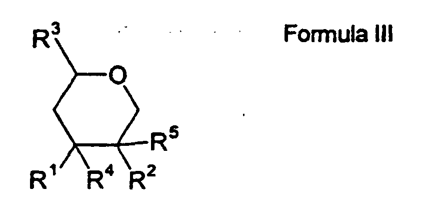 wherein R¹ and R² are independently selected from H, -OH, =O, or represent a bond with an adjacent atom on the ring of the cyclic ether;
wherein R³ is a substituent comprising an -OH group; and
wherein R⁴ and R⁵ are independently selected from H, -OH, =O or represent a bond with an adjacent atom on the ring of the cyclic ether.

Preferably the cyclic ether is a sugar.

The sugar - which may be any suitable sugar - may be naturally occurring or it may be a synthetic entity, or may be combinations thereof.

By the term "sugar" it is meant a polyhydroxy aldehyde or polyhydroxy ketone. The polyhydroxy aldehyde or polyhydroxy ketone may be optically active.

The sugar may be a monosaccharide or a oligiosaccharide. Preferably the sugar is a monosaccharide.

The sugar may be a pentose or a hexose.. Preferably the sugar is a hexose

The cyclic ether - which may be based on 1,5AnFru or a derivative thereof - may be a heterocyclic molecule comprising at least three carbon atoms, further comprising hydrogen and oxygen atoms in independently varying proportions. Examples of molecules which are based on or derived from 1,5AnFru.

Typically, the cyclic ether will comprise a heterocyclic hydrocarbyl ring. Here, the term "hydrocarbyl group" means a group comprising at least C and H and may optionally comprise one or more other suitable substituents. Examples of such substituents may include halo-, hydroxy, alkoxy-, nitro-, an alkyl group, a cyclic group etc, as well-as combinations thereof. In addition to the possibility of the substituents being a cyclic group, a combination of substituents may form a cyclic group. If the hydrocarbyl group comprises more than one C then those carbons need not necessarily be linked to each other. For example, at least two of the carbons may be linked via a suitable element or group. The hydrocarbyl group contains hetero atoms. Suitable hetero atoms will be apparent to those skilled in the art and include, for instance, sulphur, nitrogen and oxygen.

As previously mentioned, the cyclic ether has the Formula III 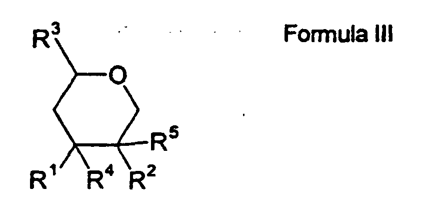 wherein R¹, R², R³, R⁴, and R⁵ are as defined above.

Preferably R³ is or comprises an -CH₂OH group.

Preferably R¹ and R² are independently selected from -OH, or =O.

Preferably at least one of R⁴ and R⁵ is H.

More preferably the cyclic ether is selected from one or more of: 1,5AnFru, or an active mimic of 1,5AnFru, a cyclic ether based on 1,5AnFru, a cyclic ether derived from 1,5AnFru - such as a tautomer or hydrate thereof, or the dehydration product of 1,5AnFru, or a tautomer or hydrate thereof, or derivatives thereof.

The cyclic ether may have a low calorie value and/or may be calorie-free, and is non-toxic.

### 1,5-Anhydro-D-fructose(1,5AnFru)

As indicated, in a preferred aspect, the cyclic ether is selected from one or more of: 1,5AnFru, or an active mimic of 1,5AnFru, a cyclic ether based on 1,5AnFru, a cyclic ether derived from 1,5AnFru - such as a tautomer or hydrate thereof, or the dehydration product of 1,5AnFru, or a tautomer or hydrate thereof, or derivatives thereof.

1,5-Anhydro-D-fructose(1,5AnFru) is a relatively inexpensive, non-toxic, low-calorie sugar. 1,5AnFru is surprisingly found to increase glucose tolerance. This effect appears to be brought about by 1,5AnFru increasing the levels of glucagon-like peptide (GLP-1) and insulin. Hence 1,5AnFru itself, or in combination with other components, is useful as a constituent of a medicament for the treatment of GLP-1 and/or insulin- related diseases.

### Production of 1,5AnFru

1,5AnFru may be formed by the action of α-1,4-glucan lyase on glycogen and related substrates, such as maltose, maltosaccharides. Alternatively, 1,5AnFru can be produced by the glucan lyase using starch as substrate (Yu *et al.,* 1999).

### Derivatives and molecules based on 1,5AnFru

Suitable derivatives of 1,5AnFru may include the isomers of 1,5AnFru which have been described chemically by Ahmad (1995), Broberg *et al*. (1998), Andersen (1999) and functionally by Yu *et al*. (1999) and Andersen *et al*. (1999).

Suitable derivatives of 1,5AnFru may also include the isomers, hydrates (for examples see Scheme 1) and dehydration products and their hydrates (for examples see Scheme 2) of 1,5AnFru, as well as 4-deoxy-glycero-hexo-2,3-diluo-furanose (Broberg *et al*., 1998), 1,5-anhydro-D-glucitol (1,5AnGlc-ol), 1,5AnGlc-ol 6-phosphate (Sakuma *et al.,* 1998; Yu *et al*. 1999) and others.

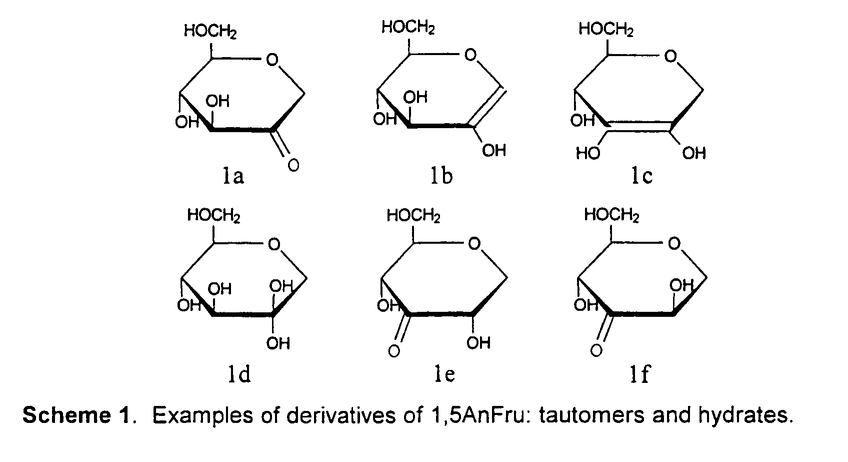

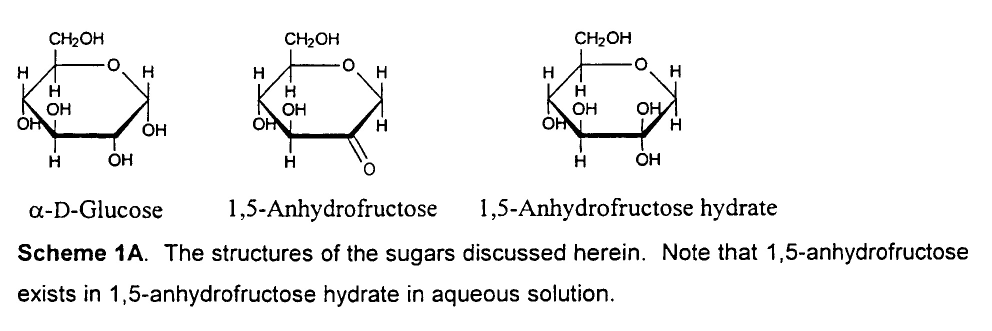

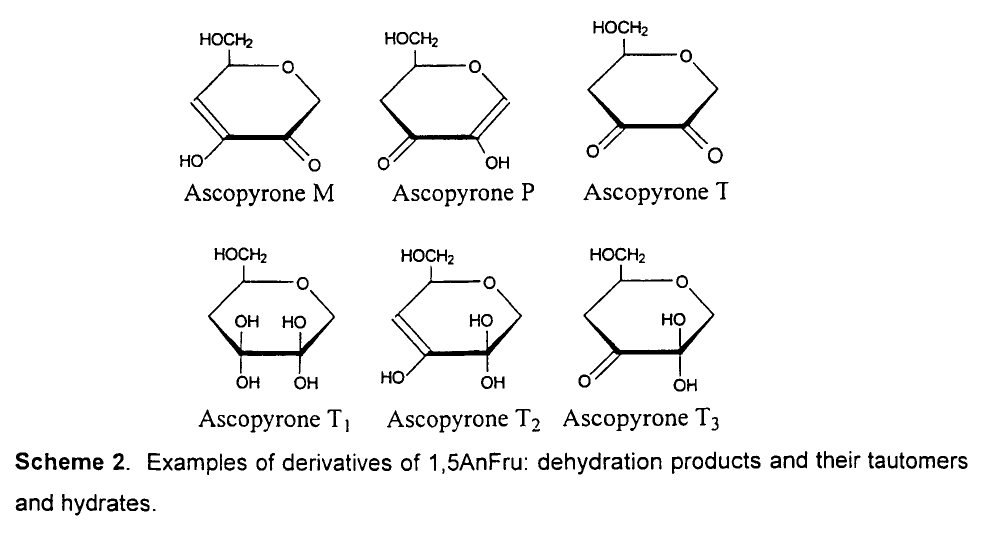

### Mimics

In one aspect, the cyclic ether can be a mimic of a 1,5AnFru structure.

The term "mimic" as used herein means having a similar or different structure but having a similar functional effect. In other words, different chemical groups or residues may comprise a similar stearic shape to a 1,5AnFru or an active part thereof.

### Pharmaceutical Form

The cyclic ether of the present invention may be in a pharmaceutically acceptable form of the cyclic ether. The cyclic ether may be in a pharmaceutically acceptable form of the cyclic ether of Formula III 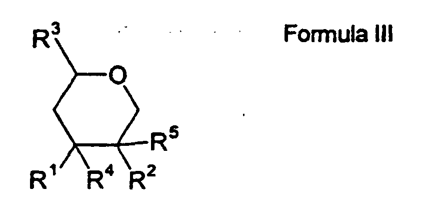 wherein R¹ and R² are independently selected from H, -OH, =O, or represent a bond with an adjacent atom on the ring of the cyclic ether;
wherein R³ is a substituent comprising an -OH group; and
wherein R⁴ and R⁵ are independently selected from H, -OH, =O or represent a bond with an adjacent atom on the ring of the cyclic ether.

The pharmaceutically acceptable form may consist of a derivative of Formula III. For example, one or more of the hydroxy groups of Formula III may be derivatised. One or more or the derivatised hydroxy groups may be an ester group.

The ester group may be an acyl ester. The ester group may be an acetyl ester. The ester group may be an esterified fatty acid.

When the compound of the present invention is 1,5AnFru, the esterified derivative may be 6-*O*-acyl-1,5-anhydro-D-fructose represented below

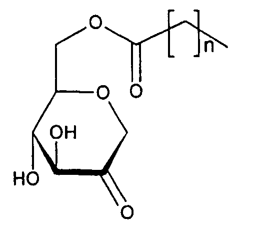

Esterified derivative in accordance with the present invention may be one or more derivative disclosed in British Patent Application No. 9906458.6, filed 19 March 1999.

The cyclic ethers of the present invention may be administered in the form of a pharmaceutically acceptable salt.

Pharmaceutically-acceptable salts are well known to those skilled in the art, and for example include those mentioned by Berge *et al*, in J.Pharm.Sci., 66, 1-19 (1977). Suitable acid addition salts are formed from acids which form non-toxic salts and include the hydrochloride, hydrobromide, hydroiodide, nitrate, sulphate, bisulphate, phosphate, hydrogenphosphate, acetate, trifluoroacetate, gluconate, lactate, salicylate, citrate, tartrate, ascorbate, succinate, maleate, fumarate, gluconate, formate, benzoate, methanesulphonate, ethanesulphonate, benzenesulphonate and p-toluenesulphonate salts.

When one or more acidic cyclic ethers are present, suitable pharmaceutically acceptable base addition salts can be formed from bases which form non-toxic salts and include the aluminium, calcium, lithium, magnesium, potassium, sodium, zinc, and pharmaceutically-active amines such as diethanolamine, salts.

The present invention also includes the use of zwitterionic forms of the cyclic ethers of the present invention.

The terms used in the claims encompass these forms.

### Stereo And Geometric Isomers

Some of the cyclic ethers may exist as stereoisomers and/or geometric isomers - e.g. they may possess one or more asymmetric and/or geometric centres and so may exist in two or more stereoisomeric and/or geometric forms. The present invention contemplates the use of all the individual stereoisomers and geometric isomers of those inhibitor agents, and mixtures thereof. The terms used in the claims encompass these forms.

### Solvates

The present invention also includes the use of solvate forms of the cyclic ethers of the present invention. The terms used in the claims encompass these forms.

### Pro-Drug

The present invention also includes the use of pro-drug forms of the cyclic ethers of the present invention. The terms used in the claims encompass these forms.

### Other Active Components

The composition of the present invention may also comprise other therapeutic substances in addition to the cyclic ether.

### Therapy

The cyclic ethers of the present invention may be used as therapeutic agents - i.e. in therapy applications.

The term "therapy" includes curative effects, alleviation effects, and prophylactic effects.

The therapy may be on humans or animals.

The therapy can include the treatment of glucose metabolism disorders, diabetes or related afflictions.

The therapy may be for treating conditions associated with altered glucose metabolism, diabetes, or chronic disease.

### Pharmaceutical Compositions

In one aspect, the present invention provides a pharmaceutical composition, which comprises a composition according to the present invention and optionally a pharmaceutically acceptable carrier, diluent or excipient (including combinations thereof).

The pharmaceutical compositions may be for human or animal usage in human and veterinary medicine and will typically comprise any one or more of a pharmaceutically acceptable diluent, carrier, or excipient. Acceptable carriers or diluents for therapeutic use are well known in the pharmaceutical art, and are described, for example, in Remington's Pharmaceutical Sciences, Mack Publishing Co. (A. R. Gennaro edit. 1985). The choice of pharmaceutical carrier, excipient or diluent can be selected with regard to the intended route of administration and standard pharmaceutical practice. The pharmaceutical compositions may comprise as - or in addition to - the carrier, excipient or diluent any suitable binder(s), lubricant(s), suspending agent(s), coating agent(s), solubilising agent(s).

Preservatives, stabilisers, dyes and even flavouring agents may be provided in the pharmaceutical composition. Examples of preservatives include sodium benzoate, sorbic acid and esters of p-hydroxybenzoic acid. Antioxidants and suspending agents may be also used.

There may be different composition/formulation requirements dependent on the different delivery systems. By way of example, the pharmaceutical composition of the present invention may be formulated to be delivered using a mini-pump or by a mucosal route, for example, as a nasal spray or aerosol for inhalation or ingestable solution, or parenterally in which the composition is formulated by an injectable form, for delivery, by, for example, an intravenous, intramuscular or subcutaneous route. Alternatively, the formulation may be designed to be delivered by both routes.

Where the agent is to be delivered mucosally through the gastrointestinal mucosa, it should be able to remain stable during transit though the gastrointestinal tract; for example, it should be resistant to proteolytic degradation, stable at acid pH and resistant to the detergent effects of bile.

Where appropriate, the pharmaceutical compositions can be administered by inhalation, in the form of a suppository or pessary, topically in the form of a lotion, solution, cream, ointment or dusting powder, by use of a skin patch, orally in the form of tablets containing excipients such as starch or lactose or chalk, or in capsules or ovules either alone or in admixture with excipients, or in the form of elixirs, solutions or suspensions containing flavouring or colouring agents, or they can be injected parenterally, for example intravenously, intramuscularly or subcutaneously. For parenteral administration, the compositions may be best used in the form of a sterile aqueous solution which may contain other substances, for example enough salts or monosaccharides to make the solution isotonic with blood. For buccal or sublingual administration the compositions may be administered in the form of tablets or lozenges which can be formulated in a conventional manner.

In the treatment of diabetes, the compound of the present invention may be used in combination with other insulinotropic agents, such as hypoglycaemic sulphonylureas, meglitinide analogues, imidazolidine, and guanidine derivatives or GLP-1 and its derivatives. Other insulinotropic agents are disclosed in W.J. Malaisse, 1999, Insulinotropic action of monosaccharide esters: therapeutic perspectives. Diabetologia, 42: 286-291.

### Administration

Typically, a physician will determine the actual dosage which will be most suitable for an individual subject and it will vary with the age, weight and response of the particular patient and severity of the condition. The dosages below are exemplary of the average case. There can, of course, be individual instances where higher or lower dosage ranges are merited.

The compositions (or component parts thereof) of the present invention may be administered orally. In addition or in the alternative the compositions (or component parts thereof) of the present invention may be administered by direct injection. In addition or in the alternative the compositions (or component parts thereof) of the present invention may be administered topically. In addition or in the alternative the compositions (or component parts thereof) of the present invention may be administered by inhalation. In addition or in the alternative the compositions (or component parts thereof) of the present invention may also be administered by one or more of: parenteral, mucosal, intramuscular, intravenous, subcutaneous, intraocular or transdermal administration means, and are formulated for such administration.

Depending upon the need, the agent may be administered at a dose of from 0.0001 to 3000 mg/kg body weight, such as from 0.01 to 100 mg/kg, more preferably from 0.1 to 1 mg/kg body weight.

By way of further example, the agents of the present invention may be administered in accordance with a regimen of 1 to 10 times per day, such as once or twice per day. The specific dose level and frequency of dosage for any particular patient may be varied and will depend upon a variety of factors including the activity of the specific compound employed, the metabolic stability and length of action of that compound, the age, body weight, general health, sex, diet, mode and time of administration, rate of excretion, drug combination, the severity of the particular condition, and the host undergoing therapy.

The term "administered" also includes but is not limited to delivery by a mucosal route, for example, as a nasal spray or aerosol for inhalation or as an ingestable solution; a parenteral route where delivery is by an injectable form, such as, for example, an intravenous, intramuscular or subcutaneous route.

Hence, the cyclic ether of the present invention may be administered by one or more of the following routes: oral administration, injection (such as direct injection), topical, inhalation, parenteral administration, mucosal administration, intramuscular administration, intravenous administration, subcutaneous administration, intraocular administration or transdermal administration.

For some applications, preferably the agent is administered orally.

Here, the cyclic ether (or its derivatives) is given in either liquid or solid form or mixed with other suitable components. It may be dissolved in soft drinks or added in selected food products.

Thus, preferably, the composition of the present invention is administered orally for treatment of glucose metabolism disorders.

### 1,5AnFru and Sugar Metabolism

In fungi and algae, 1,5AnFru may be converted to antibiotics under stress conditions (Baute *et al*., 1988). (Baute *et al*., 1998; Broberg *et al.,* 1999), while in mammals and *E. coli* 1,5AnFru is reduced by NADPH-dependent reductase to 1,5-anhydroglucitol (Sakuma *et al.,* 1998; Yu *et al*., 1999). This polyol may be phosphorylated or filtered directly into the preurine, where it competes with glucose for tubular re-absorption (Yamanouchi *et al*., 1992). Low plasma levels of 1,5-anhydro glucitol have been shown to correlate to glucosuria in diabetics, and plasma 1,5-anhydro glucitol has been suggested to be a marker for the glycemic control in diabetes (Yamanouchi *et al*., 1989; Stickle and Turk, 1997). The main portion of plasma 1,5-anhydro glucitol is derived from dietary intake and only a small fraction is derived from in vivo reduction of 1,5-anhydro fructose (Yamanouchi *et al*., 1992).

Since it represents an alternative degradation pathway of glycogen, we examined its influence on insulin secretion and glucose disposal in mice. We found that at appropriate dose levels, 1,5AnFru inhibited glucose-stimulated insulin secretion during an intravenous glucose tolerance test *in vivo* and after raising the glucose level in the incubation medium when isolated islets were incubated *in vitro*.

Inhibition of insulin secretion from islets may be due to inhibition of glucokinase and hexokinase by 1,5AnFru (or derivatives thereof) according to the invention.

It is shown herein that gastric administration of 1,5AnFru potentiates insulin secretion and glucose tolerance during gastric glucose tolerance test.

The plasma levels of GLP-1 in the mice after gastric glucose are examined, GLP-1 being a gut hormone released by enteral glucose acting as an incretin hormone potentiating insulin secretion (Ahrén, 1998). It is found that 1,5AnFru markedly enhances the GLP-1 response to gastric glucose. It is therefore possible that the augmented insulin response by gastric 1,5AnFru is mediated by the increased levels of GLP-1. 1,5AnFru (or derivatives thereof) may therefore stimulate GLP-1 secretion from the intestinal L-cells.

Glucose may activate GLP-1 secretion from intestinal L cells through absorption from the luminal side (Sugiyama *et al*., 1994). 1,5AnFru may delay the absorption of glucose in the gut, enabling more glucose to reach the L-cells, in analogy with the pseudotetrasaccharide, acarbose, which increases GLP-1 secretion after oral sucrose by inhibiting enteral α-glucosidase, thereby postponing gut absorption of glucose to more distal parts of the gut with a higher L-cell density (Seifarth *et al*., 1998). 1,5AnFru (or derivatives thereof) may directly stimulate GLP-1 secretion from the intestinal L-cells.

GLP-1 secretion from the L-cells is governed by a sodium-glucose co-transporter mechanism, and carbohydrates that activate this mechanism, like glucose, galactose, methyl-α-glucoside and 3-O-methyl glucose, stimulate GLP-1 secretion, whereas carbohydrates which are not substrates for this luminal sodium/glucose transport, like 2-de-oxy-glucose and N-acetyl-glucosamine, do not stimulate GLP-1 secretion (Ritzel *et al*., 1997). In addition, a sodium-independent GLP-1 secretion has also been found since fructose stimulates GLP-1 secretion independently from sodium (Ritzel *et al.,* 1997).

1,5AnFru (or derivatives thereof) may therefore be a substrate for the sodium/glucose transport mechanism and therefore activate GLP-1 secretion through this pathway or, like fructose, may do so through a sodium-independent mechanism.

The cyclic ether of the present invention may be advantageously employed to augment endogenous GLP-1 secretion. This would augment insulin secretion after meal intake (Nauck *et al*., 1997). This would be of particular interest in view of the reduction of GLP-1 secretion after meal which is often seen in diabetics (Toft-Nielsen *et al*., 1999). The present invention therefore provides a method for augmenting endogenous GLP-1 secretion and improving glucose tolerance after gastric administration of glucose by administration of compositions according to the present invention.

It is known that a wide of range carbohydrates can stimulate secretion of GLP-1 and the possible mechanism may be the interaction of these carbohydrates with a uncharacterised sugar-sensor, which triggers the L-cells to produce GLP-1, which in turn interacts with the GLP-1 acceptor on the cells that produce insulin (Shima *et al*., 1990; Ritzel *et al*., 1997).

Due to the severe chemical and biological conditions in the intestine, we believe (without wishing to be bound by theory) that 1,5AnFru can be converted to different isomers and derivatives (Scheme 1 and 2). We believe that at least most of them should have interaction with the sugar-sensor according to the model proposed (Shima *et al*. 1990; Ritzel *et al*., 1997) that requires a hexose ring.

### Summary

The use of 1,5-AnFru (or derivatives thereof) advantageously increases glucose tolerance by increasing GLP-1 and insulin.

Thus, according to the present invention, a cyclic ether and its derivatives may be advantageously used to increase glucose tolerance by increasing glucagon-like peptide 1 (GLP-1) and insulin, and are therefore useful as medicament(s) for improving the condition of patients suffering from diseases related to glucose intolerance or to GLP-1 or insulin.

### Examples

The present invention will now be described by way of example, in which reference is made to the accompanying Figures:
Figure 1 which is a graph
Figure 2 which is a graph
Figure 3 which is a graph
Figure 4 which is a graph

In more detail:

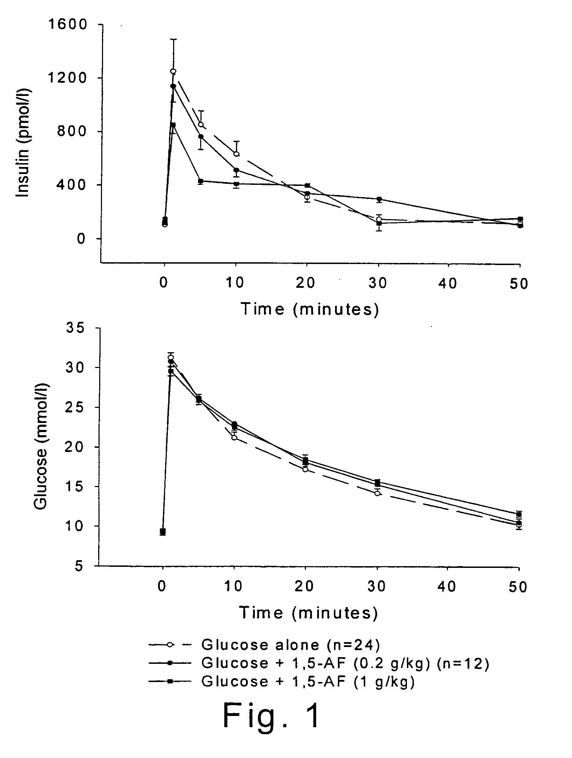

**Fig. 1** Plasma insulin and glucose levels immediately before and at 1, 5, 10, 20, 30 and 50 min after an iv injection of glucose (1 g/kg) with or without addition of 1,5AnFru at 0.2 or 1 g/kg in anesthetized mice. Means±SEM are shown. n indicates number of mice in each group.

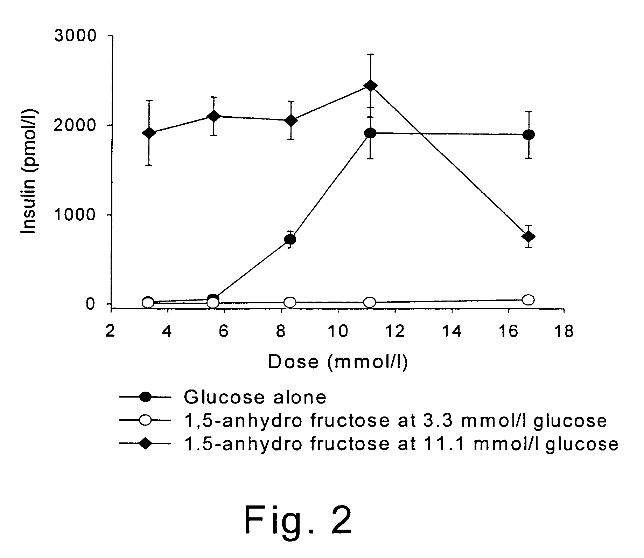

**Fig. 2** Insulin secretion from overnight cultured isolated mouse islets during a 60 min incubation in presence of different concentrations of glucose or 1,5AnFru (at 3.3 or 11.1 mmol/l glucose). Values are mean±SEM. There were 24 observations in each point.

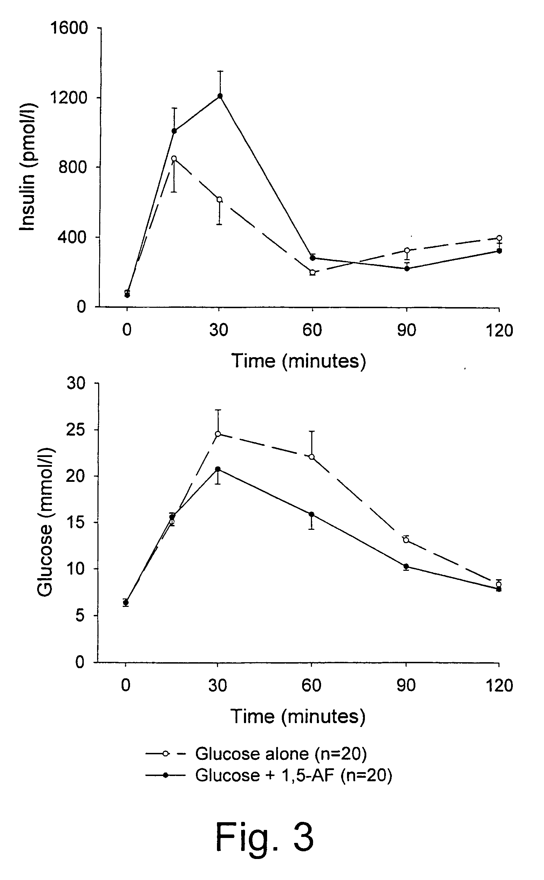

**Fig. 3** Plasma insulin and glucose immediately before and at 15, 30, 60, 90 and 120 min after administration of glucose (150 mg/mouse) through a gastric gavage with or without addition of 1,5AnFru (150 mg/mouse) in anesthetized mice. Means±SEM are shown. n indicates number of mice in each group.

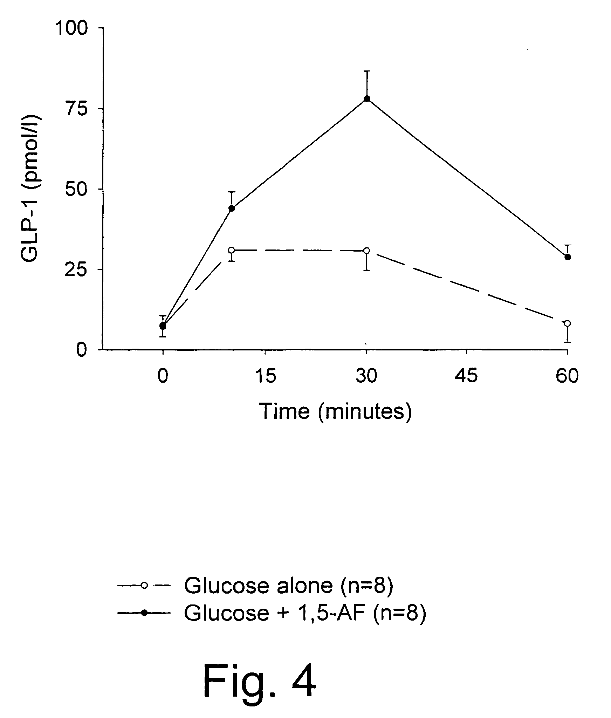

**Fig. 4** Plasma GLP-1 immediately before and at 15, 30 and 60 min after administration of glucose (150 mg/mouse) through a gastric gavage with or without addition of 1,5AnFru (150 mg/mouse) in anesthetized mice. Means±SEM are shown. n indicates number of mice in each group.

### General Methods

### Animals.

Non-fasted NMRI mice (Bomholdtgaard Breeding and Research Center, Ry, Denmark), weighing 20-25 g are used throughout the study. The animals are fed a standard pellet diet and tap water ad libitum.

### Intravenous glucose tolerance test.

The mice are anesthetized with an intraperitoneal injection of midazolam (Dormicum^{R}, Hoffman-La-Roche, Basel, Switzerland, 0.4 mg/mouse) and a combination of fluanison (0.9 mg/mouse) and fentanyl (0.02 mg/mouse; Hypnorm^{R}, Janssen, Beerse, Belgium). Thereafter, a blood sample is taken from the retrobulbar, intraorbital, capillary plexus in heparinized tubes, whereafter D-glucose (British Drug Houses, Poole, UK; 1 g/kg) is injected rapidly intravenously either alone or together with 1,5AnFru (Danisco Ltd, Copenhagen, Denmark; 0.2 or 1 g/kg); in one series of experiments, 1,5AnFru is given alone (1 g/kg). The volume load is 10 µl/g body weight. New blood samples are taken after 1, 5, 10, 20, 30 and 50 minutes. In another set of experiments, 1,5AnFru is given by gastric gavage (0.2 g/kg) five minutes before the zero blood sample taken immediately before the intravenous injection of glucose as above. Blood samples are taken as above. Following immediate centrifugation at 4°C, plasma is separated and stored at -20°C or until analysis.

### Gastric glucose tolerance test.

The mice are fasted overnight and anesthetized as above. After induction of anesthesia, D-glucose (150 mg/mouse in 0.5 ml) is administered alone or together with 1,5AnFru (150 mg/mouse) through a gavage tube (outer diameter 1.2 mm) placed in the stomach. Blood samples are taken after 15, 30, 45, 60, 90 and 120 minutes and treated as above.

### Insulin secretion in vitro.

Pancreatic islets are isolated from four mice with the collagenase isolation technique. In brief, the pancreas is filled retrogradely through the pancreatic duct with 3 ml of Hank's Balanced Salt Solution (Sigma), supplemented with 0.3 mg/ml of Collagenase P (activity 1.86 U/mg; Boehringer Mannheim Gmbh, Mannheim, Germany). The pancreas is subsequently removed and incubated in the same solution for 20 min at 37°C. After rinsing, the islets are handpicked under a stereomicroscope and incubated overnight in RPMI 1640 medium supplemented with 10% fetal calf serum, 2.05 mmol/l L-glutamine, 2.5 µg/ml amphotericin B (GIBCO BRL, Paisley, Scotland), 100 IU/ml penicillin and 100 µg/ml streptomycin (Biol Ind, Beit Haemek, Israel) at 37°C in humidified air equilibrated with 5% CO₂. Following the overnight incubation, the islets are washed three times and then preincubated for 60 min at 37°C in a Hepes medium (pH 7.36) supplemented with 0.1% human serum albumin (Sigma) and 3.3 mmol/l glucose. The medium consists of (in mmol/l): 125 NaCl, 5.9 KCI, 1.2 MgCl₂, 1.28 CaCl₂ (all Sigma) and 25 Hepes (Boehringer Mannheim). After the pre-incubation, groups of three islets are transferred into separate chambers containing 200 µl of the medium supplemented with glucose and 1,5AnFru at various concentrations. Following incubation at 37°C for 60 min, 25 µl of the medium is collected from each chamber and stored at -20°C until analysis.

### Analysis.

Plasma insulin is determined radioimmunochemically with the use of a guinea pig anti-rat insulin antibody, ¹²⁵I-labelled porcine insulin as tracer and rat insulin as standard (Linco Research, St Charles, Mo, USA). Free and bound radioactivity is separated by use of an anti-IgG (goat anti-guinea pig) antibody (Linco). The sensitivity of the assay is 12 pmol/l and the coefficiency of variation is less than 3% at both low and high levels. Plasma glucose is determined with the glucose oxidase method. Plasma GLP-1 is measured by a radioimmunoassay after extraction of plasma samples with ethanol. 400 µl 0.05 mol/l sodium phosphate buffer, pH 7.5, containing 6% albumin and 0.1 mol/l NaCl is added to 100 µl mouse plasma on ice and mixed well. The mixture is then extracted with 70% ethanol (vol/vol, final dilution), and after vacuum centrifugation the residue is reconstituted in assay buffer and assayed as previously described (Ørskov *et al*., 1994). The antiserum (code no. 89390) is highly specific for C-terminal intestinal GLP-1, and recognizes mouse GLP-1. The sensitivity using this procedure is 5 pmol/l, and the intra-assay coefficient of variation is 10%. The recovery of GLP-1 added to mouse plasma is within ±20% of expected values.

### Statistical analysis.

Means±SEM are shown. Area under the curve for plasma insulin levels (AUCᵢₙₛᵤₗᵢₙ) is calculated by the trapezoid rule. Statistical analyses are performed with the SPSS for Windows system. Statistical comparisons between groups are performed with Students t-test.

### Intravenous glucose tolerance test.

Fig. 1 shows that basal plasma levels of insulin or glucose are not affected by 1,5AnFru when the sugar was given alone (1 g/kg). However, when given together with glucose (1 g/kg), 1,5AnFru inhibites glucose-stimulated insulin secretion when given at 1 g/kg. Thus, the area under the insulin curve during the 50 min study period, AUCᵢₙₛᵤₗᵢₙ, which was 14.4±2.1 nmol/l x 50 min in the controls given glucose alone and 14.6±1.9 nmol/l x 50 min in mice given glucose and 1,5AnFru at 0.2 g/kg, is reduced to 8.6±1.9 in mice given glucose and 1,5AnFru at 1 g/kg (P=0.021). In contrast, glucose elimination after the intravenous glucose administration is not affected by 1,5AnFru.

### Insulin secretion in vitro

Fig. 2 shows that 1,5AnFru does not affect glucose-stimulated insulin secretion when added to isolated mouse islets at dose levels of 11.1 mmol/l or below. However, when a high dose level of 16.7 mmol/l 1,5AnFru is added together with 11.1 mmol/l glucose, inhibition of insulin secretion is observed.

### Gastric glucose tolerance test

When 1,5AnFru is given through a gastric gavage together with glucose, the plasma insulin levels are increased in comparison when glucose is given alone (Fig. 3). Thus, the AUCᵢₙₛᵤₗᵢₙ during the first 60 min after administration is increased by 1,5AnFru from 20.3±2.3 nmol/l in 60 min in controls to 32.9±2.6 nmol/l in 60 min in mice given glucose and 1,5AnFru (P=0.018). This is followed by increased glucose elimination, as evidenced by higher 60 min glucose value in the control group (22.1±2.8 mmol/l) than in mice given glucose with 1,5AnFru (15.5±1.6 mmol/l; P=0.021).

### Plasma GLP-1 after gastric gavage

Adminstration of glucose through gastric gavage increases plasma levels of GLP-1. The increase in GLP-1 levels is potentiated by the combined aministration of 1,5AnFru and glucose (Fig. 4). Thus, both the 30 min (30.8±6.1 versus 78.2±8.6 pmol/l) and the 60 min (8.2±5.9 versus 28.8±3.8 pmol/l) values are higher after administration of glucose with 1,5AnFru than after administration of glucose alone (P<0.05 for both).

### The influence of 1,5AnFru on glucose-stimulated insulin secretion both in vivo and in vitro is examined.

*In vivo,* the influence of the sugar on insulin secretion and glucose tolerance is determined both during an intravenous glucose tolerance test and when giving 1,5AnFru together with glucose through gastric gavage in anesthetized mice.

It is found that when administered intravenously at 1 g/kg, 1,5AnFru inhibits the insulin response to iv glucose (1 g/kg), without affecting the glucose elimination during the 50 min study period.

When incubated with isolated islets, 1,5AnFru at 16.7 mmol/l, inhibits glucose (11.1 mM)-stimulated insulin secretion. Furthermore, when given through a gastric gavage (150 mg/mouse) together with glucose (150 mg/mouse), 1,5AnFru increases glucose tolerance, as evident by reduced 60 min plasma glucose level (15.5±1.6 mmol/l versus 22.1±2.8 mmol/l in controls; P=0.021). Simultaneously, insulin secretion is increased by 1,5AnFru (AUCᵢₙₛᵤₗᵢₙ during 60 min was 32.9±2.6 versus 20.3±2.3 nmol/l in control, P=0.018).

Furthermore, 1,5AnFru potentiates the increase in plasma levels of the gut hormone, glucagon-like peptide-1 (GLP-1) when given through the gastric gavage. We disclose herein that 1,5AnFru given enterally increases glucose tolerance in mice by increasing insulin secretion due to increased plasma levels of GLP-1.

All publications mentioned in the above specification are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in chemistry, biochemistry, biotechnology or related fields are intended to be within the scope of the following claims.

### References - Section 1

Ahrén, B., 1998. Glucagon-like peptide 1 (GLP-1) - a gut hormone of potential interest in the treatment of diabetes. BioEssays 20,642-651.
Baute, M.A., Baute, R., Deffieux, G. 1988. Fungal enzyme activity degrading 1,4- - glucans to 1,5-anhydro-D-fructose. Phytochemistry 27, 3401-3403.
Broberg, A., Kenne L., Pedersén, M. 1999. Analysis of 1,5-anhydro-d-fructose, microthecin, and 4-deoxy-*glycero*-hexo-2,3-diulose in algae using gas chromatography-mass spectrometry in selected ion monitoring mode. Anal. Biochem. 268,35-42.
Deacon, C.F., Nauck, M.A., Toft-Nielsen, M., Pridal, L., Willms, B., Holst, J.J. 1995. Both subcutaneously and intravenously administered glucagon-like peptide I are rapidly degraded from the NH₂-terminus in type II diabetic patients and in healthy subjects. Diabetes 44,1126-1131.
Deacon, C.F., Knudsen, L.B., Madsen, K., Wiberg, F.C., Jacobsen, O., Holst, J.J. 1998. Dipeptidyl peptidase IV resistant analogues of glucagon-oike peptide-1 which have extended metabolic stability and improved biological activity. Diabetologia 41,271-278.
Gutniak, M.K., Larsson, H., Saunders, S.W., Juneskans, O., Holst, J.J., Ahrén, B., 1997. GLP-1 tablet in NIDDM in fasting and postprandial conditions. Diabetes Care 20,1874-1879.
Holst, J.J., Deacon, C., Toft-Nielsen, M.B., Bjerre Knudsen, L., 1998. On the treatment of diabetes mellitus with glucagon-like peptide-1. Ann. N.Y. Acad. Sci. 865,336-343.
Holst, J.J., Deacon, C.F., 1988. Inhibition of the activity of dipeptidyl-peptidase IV as a treatment for type 2 diabetes. Diabetes 47,1663-1670.
Kametani, S., Shiga, Y., Akanuma, H. 1996. Hepatic production of 1,5-anhydrofructose and 1,5-anhydroglucitol in rat by the third glycogenolytic pathway. Eur. J. Biochem. 242,832-838.
Larner, J., 1990. Insulin and the stimulation of glycogen synthesis. The road from glycogen structure to glycogen synthase to cyclic AMP-dependent protein kinase to insulin mediators. In: Advances in Enzymology and Related Areas of Molecular Biology (Meister A, ed.), Jwiley and Sones, New York, pp 173-231
Nauck, M.A., 1998. Glucagon-like peptide 1 (GLP-1): a potent gut hormone with a possible therapeutic perspective. Acta Diabetol. 35, 117-129.
Nauck, M.A., Holst, J.J., Willms, B., Schmiegel, W., 1997. Glucagon-like peptide 1 (GLP-1) as a new therapeutic approach for type 2-diabetes. Exp. Clin. Endocrinol. Diabetes 105,187-195.
Printen, J.A., Brady, M.J., Saltiel, A.R. 1997. PTG, a protein phosphatase 1-binging protein with a role in glycogen metabolism. Science 275,475-1478
Ritzel, U., Fromme, A., Ottleben, M., Leonhardt, U., Ramadori, G., 1997. Release of glucagon-like peptide-1 (GLP-1) by carbohydrates in the perfused rat ileum. Acta Diabetol. 34,18-21.
Sakuma, M., Kametani, S., Akanuma, H., 1998. Purification and some properties of a hepatic NADPH-dependent reductase that specifically acts on 1,5-anhydro-D-fructose. J. Biochem. 123,189-193.
Seifarth, C., Bergmann, J., Holst, J.J., Ritzel, R., Schmiegel, W., Nauck, M.A., 1998. Prolonged and enhanced secretion of glucagon-like peptide 1 (7-36 amide) after oral sucrose due to alpha-glucosidase inhibition (acarbose) in type 2 diabetic patients. Diabet. Med. 15,485-491.
Shiga, Y., Kametani, S., Kadokura, T., Akanuma, H. 1999. 1,5-Anhydroglucitol promotes glycogenolydsis in *Escherichia coli.* J. Biochem. 125,166-172
Stickle, D., Turk, J., 1997. A kinetic mass balance model for 1,5-anhydroglucitol: applications to monitoring of glycemic control. Am. J. Physiol. 273,E821-E830.
Sugiyama, K., Manaka, H., Kato, T., Yamatani, K., Tominaga, M., Sasaki, H., 1994. Stimulation of truncated glucagon-like peptide-1 release from the isolated perfused canine ileum by glucose absorption. Digestion 55,24-28.
Taguchi, T., Haruna, M., Okuda, J., 1993. Effects of 1,5-anhydro-D-fructose on selected glucose-metabolizing enzymes. Biotechnol. Appl. Biochem. 18,275-283.
Toft-Nielsen, M., Damholt, M.B., Hilsted, L., Hughes, T.E., Krarup, T., Madsbad, S., Holst, J.J., 1999. GLP-1 secretion is decreased in NIDDM patients compared to matched control subjecys with normal glucose tolerance. Diabetologia 42:supplement 1,A40.
Yamanouchi, T., Minoda, S., Yabuchi, M., Akanuma, Y., Akanuma, H., Miyashita, H., Akaoka, I., 1989. Plasma 1,5-anhydro-D-glucitol as new clinical marker of glycemic control in NIDDM patients. Diabetes 38,723-729.
Yamanouchi, T., Tachibana, Y., Akanuma, H., Minoda, S., Shinohara, T., Moromizato, H., Miyashita, H., Akaoka, I., 1992. Origin and disoposal of 1,5-anhydroglucitol, a major polyol in the human body. Am. J. Physiol. 263,E268-E273.
Yu, S., Bojsen, K., Svensson, B., Marcussen, K., 1999. α-1,4-glucan lyases producing 1,5-anhydro-D-fructose rom starch and glycogen have sequence similarity to α-glucosidases. Biochim Biophys. Acta 1433,1-15.
Ørskov, C., Rabenhøj, L., Kofod, H., Wettergren, A., Holst, J.J., 1994. Production and secretion of amidated and glycine-extended glucagon-oike peptide-1 (GLP-1) in man. Diabetes 43,535-539.

### References - Section 2

1. Mathews, C. K. and van Holde K. E. 1990. In: Biochemistry (edited by Mathews, C. K. and van Holde K. E, pp. 460-466; 555-556; . The Benjamin/Cummings Publishing Company, Redwood City, CA, USA.
2. Yu, S. Bojsen, K., Svensson, B., and Marcussen, J.(1999) α-1,4-Glucan lyases producing 1,5-anhydro-D-fructose from starch and glycogen have sequence similarity to α-glucosidases. Biochim. Biophys. Acta. 1433: 1-15.
3. Shiga, Y., Kametani, S., Kadokura, T., and Akanuma, H. (1999) 1,5-Anhydroglucitol promotes glycogenolydsis in *Escherichia coli.* J. Biochem. 125: 166-172.
4. Yamanouchi, T., Minoda, S., Yabuchi, M., Akanuma, Y., Akanuma, H., Miyashita, H., Akaoka, I., (1989). Plasma 1,5-anhydro-D-glucitol as new clinical marker of glycemic control in NIDDM patients. Diabetes 38: 723-729.
5. Shima, K., Suda, T., Nishimoto, K., Yoshimoto, S.. (1997) Relationship between molecular structures of sugars and their ability to stimulate the release of glucagon-like peptide-1 from canine ileal loops. Acta Endocrinol (Copenh). 123: 464-470.
6. Ritzel, U., Fromme, A., Ottleben, M., Leonhardt, U., Ramadori, G., (1997). Release of glucagon-like peptide-1 (GLP-1) by carbohydrates in the perfused rat ileum. Acta Diabetol. 34: 18-21.
7. Ahmad, T. , (1995) *Studies on the degradation of some pentoses and of 1,5-anhydro-D-fructose, the product of the starch-degrading enzyme α-1,4-glucan lyase.* PhD thesis, The Swedish University of Agricultural Sciences.
8. Broberg, A. , (1998) *Structural and quantitative studies of metabolites in red algae.* PhD. thesis, The Swedish University of Agricultural Sciences.
9. Andersen, M. A., (1999). *1,5-Anhydro-D-fructose, structure, characterisation and derivatives.* PhD thesis. The technical University of Denmark.
10. Yu, S., Andersen, M. S., Jensen, H. M., Isak, T., and Marcussen, J. (1999). *Further modification of A.F. (ascopyrone).* UK application filed on March 19, 1999.
11. Andersen, M. S., Yu, S., Lundt, I., and Marcussen, J. (1999), *Bifunctional ingredient* .UK application filed on March 19, 1999.
12. Broberg, A., Kenne, L., and Pedersén, M. (1998). Formation of 4-deoxy-glycero-hexo-2,3-diluo-furanose. Carbohydr. Res. 306: 171-175.
13. Sakuma, M., Kametani, S., and Akanuma, H. (1998) Purification and some properties of a hepatic NADPH-dependent reductase that specifically acts on 1,5-anhydro-D-fructose, J. Biochem. 123: 189-193.

### REFERENCES To GLP-1 Section:

1. Dillon, J. S.; Tanizawa, Y.; Wheeler, M. B.; Leng, X.-H.; Ligon, B. B.; Rabin, D. U.; Yoo-Warren, H.; Permutt, M. A.; Boyd, A. E., III : Cloning and functional expression of the human glucagon-like peptide-1 (GLP-1) receptor. Endocrinology 133: 1907-1910, 1993. PubMed ID : 8404634
2. Kershaw, E. E.; Chua, S. C., Jr.; Leibel, R. L. : Localization of a (CA)n repeat in glucagon-like peptide-1 receptor gene (Glp1r) to proximal mouse chromosome 17 and its linkage to other markers. Mammalian Genome 6: 301-303, 1995. PubMed ID : 7613039
3. Stoffel, M.; Espinosa, R., III; Le Beau, M. M.; Bell, G. I. : Human glucagon-like peptide-1 receptor gene: localization to chromosome band 6p21 by fluorescence in situ hybridization and linkage of a highly polymorphic simple tandem repeat DNA polymorphism to other markers on chromosome 6. Diabetes 42: 1215-1218, 1993. PubMed ID : 8392011
4. Thorens, B. : Expression cloning of the pancreatic beta cell receptor for the gluco-incretin hormone glucagon-like peptide 1. Proc. Nat. Acad. Sci. 89: 8641-8645, 1992. PubMed ID : 1326760

## Claims

1. Use of a cyclic ether, or a pharmaceutically acceptable form (e.g. salt) thereof, in the manufacture of a medicament to treat a glucose tolerance disorder wherein the cyclic ether has the Formula III: 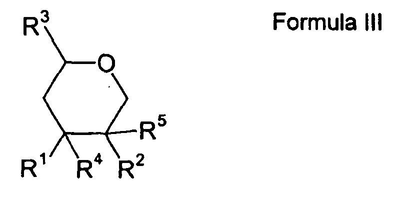 wherein R¹ and R² are independently selected from H, -OH, =O, or represent a bond with an adjacent atom on the ring of the cyclic ether;
wherein R³ is a substituent comprising an -OH group; and
wherein R⁴ and R⁵ are independently selected from H, -OH, =O or represent a bond with an adjacent atom on the ring of the cyclic ether.

2. Use according to claim 1 wherein the cyclic ether is a sugar.

3. Use according to claim 1 or 2 wherein R³ is or comprises an -CH₂OH group.

4. Use according to any one of claims 1 to 3 wherein R¹ and R² are independently selected from -OH, or =O.

5. Use according to any one of claims 1 to 4 wherein at least one of R⁴ and R⁵ is H.

6. Use according to any one of the preceding claims wherein said cyclic ether is selected from one or more of: 1,5 anhydrofructose ("1,5AnFru"), or an active mimic of 1,5AnFru, a cyclic ether based on 1,5AnFru, a cyclic ether derived from 1,5AnFru - such as a tautomer or hydrate thereof, or the dehydration product of 1,5AnFru, or a tautomer or hydrate thereof, or derivatives thereof.

7. Use according to any preceding claim wherein said cyclic ether is 1,5AnFru.

8. Use according to any preceding claim wherein said medicament is to treat diabetes.

9. A composition for use in medicine, said composition comprising:
i) a cyclic ether as an active ingredient, wherein said cyclic ether is the cyclic ether as defined in any one of claims 1 to 8; and optionally
ii) a pharmaceutically acceptable carrier, diluent or excipient;
wherein said cyclic ether is capable of affecting glucose tolerance.

10. A composition according to claim 9 wherein said composition is for modulating GLP-1 and/or treating a glucose tolerance disorder, such as diabetes.

11. A process for preparing a composition as defined in any one of claims 9 to 10, said process comprising the steps of:
i) providing a cyclic ether as defined in any one of claims 1 to 8; and
ii) admixing said cyclic ether with a pharmaceutically acceptable carrier, diluent or excipient.

12. A process according to claim 11 wherein said composition is to be used for affecting glucose tolerance disorder and/or modulating GLP-1.

13. Use of 1,5AnFru in the manufacture of a medicament for affecting glucose tolerance disorder.

## Patentansprüche

1. Verwendung eines zyklischen Ethers oder einer pharmazeutisch verträglichen Form (z.B. Salz) davon bei der Herstellung eines Medikaments zur Behandlung einer Glucosetoleranzstörung, wobei der zyklische Ether die Formel III hat: 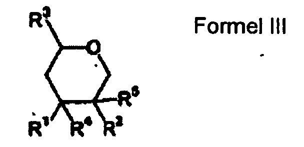 wobei R¹ und R² unabhängig voneinander unter H, -OH und =O ausgewählt sind oder eine Bindung mit einem benachbarten Atom an dem Ring des zyklischen Ethers darstellen,
wobei R³ ein Substituent ist, der eine OH-Gruppe enthält, und
wobei R⁴ und R⁵ unabhängig voneinander unter H, -OH und =O ausgewählt sind oder eine Bindung mit einem benachbarten Atom an dem Ring des zyklischen Ethers darstellen.

2. Verwendung nach Anspruch 1, wobei der zyklische Ether ein Zucker ist.

3. Verwendung nach Anspruch 1 oder 2, wobei R³ eine -CH₂OH-Gruppe ist oder enthält.

4. Verwendung nach einem der Ansprüche 1 bis 3, wobei R¹ und R² unabhängig voneinander unter -OH oder =O ausgewählt sind.

5. Verwendung nach einem der Ansprüche 1 bis 4, wobei wenigstens einer von R⁴ und R⁵ H ist.

6. Verwendung nach einem der vorangegangenen Ansprüche, wobei der zyklische Ether unter einem oder mehreren der folgenden ausgewählt ist: 1,5-Anhydrofructose ("1,5AnFru") oder einem aktiven Nachahmer von 1,5AnFru, einem zyklischen Ether auf der Grundlage von 1,5AnFru, einem zyklischen Ether, der von 1,5AnFru abgeleitet ist, wie einem Tautomer oder einem Hydrat davon, oder dem Dehydratisierungsprodukt von 1,5AnFru oder einem Tautomer oder Hydrat davon oder von Derivaten davon.

7. Verwendung nach einem der vorangegangenen Ansprüche, wobei der zyklische Ether 1,5AnFru ist.

8. Verwendung nach einem der vorangegangenen Ansprüche, wobei das Medikament für die Behandlung von Diabetes vorgesehen ist.

9. Zusammensetzung für eine Verwendung in der Medizin, wobei die Zusammensetzung folgendes umfaßt:
i) einen zyklischen Ether als einen aktiven Bestandteil, wobei der zyklische Ether der zyklische Ether ist, wie er in einem der Ansprüche 1 bis 8 definiert ist, und optional
ii) einen pharmazeutisch verträglichen Träger, ein Verdünnungsmittel oder ein Bindemittel,
wobei der zyklische Ether in der Lage ist, Glucosetoleranz zu beeinflussen.

10. Zusammensetzung nach Anspruch 9, wobei die Zusammensetzung für das Modulieren von GLP-1 und/oder zur Behandlung einer Glucosetoleranzstörung, wie beispielsweise Diabetes, vorgesehen ist.

11. Verfahren zur Herstellung einer Zusammensetzung, wie sie in einem der Ansprüche 9 bis 10 definiert ist, wobei das Verfahren die Stufen umfaßt, in denen man
i) einen zyklischen Ether bereitstellt, wie er in einem der Ansprüche 1 bis 8 definiert ist, und
ii) den zyklischen Ether mit einem pharmazeutisch verträglichen Träger, Verdünnungsmittel oder Bindemittel mischt.

12. Verfahren nach Anspruch 11, wobei die Zusammensetzung für eine Beeinflussung von Glucosetoleranzstörung und/oder zum Modulieren von GLP-1 zu verwenden ist.

13. Verwendung von 1,5AnFru bei der Herstellung eines Medikaments zur Beeinflussung von Glucosetoleranzstörung.

## Revendications

1. Utilisation d'un éther cyclique, ou d'une de ses formes pharmaceutiquement acceptables (par exemple un sel), dans la production d'un médicament destiné au traitement d'un trouble de tolérance au glucose, dans laquelle l'éther cyclique répond à la formule III : 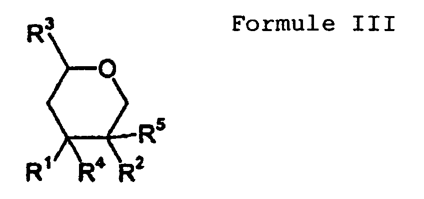 dans laquelle R¹ et R² sont choisis indépendamment entre H, un groupe -OH, un groupe =O, ou bien représentent une liaison avec un atome adjacent sur le noyau de l'éther cyclique ;
dans laquelle R³ représente un substituant comprenant un groupe -OH ; et
dans laquelle R⁴ et R⁵ sont choisis indépendamment entre H, un groupe -OH, un groupe =O, ou représentent une liaison avec un atome adjacent sur le noyau de l'éther cyclique.

2. Utilisation suivant la revendication 1, dans laquelle l'éther cyclique est un sucre.

3. Utilisation suivant la revendication 1 ou 2, dans laquelle R³ est ou comprend un groupe -CH₂OH.

4. Utilisation suivant l'une quelconque des revendications 1 à 3, dans laquelle R¹ et R² sont choisis indépendamment entre un groupe -OH et un groupe =O.

5. Utilisation suivant l'une quelconque des revendications 1 à 4, dans laquelle au moins un des groupes R⁴ et R⁵ représente H.

6. Utilisation suivant l'une quelconque des revendications précédentes, dans laquelle ledit éther cyclique choisi est un ou plusieurs des suivants : 1,5-anhydrofructose ("1,5AnFru"), ou un agent mimétique actif de 1,5AnFru, un éther cyclique à base de 1,5AnFru, un éther cyclique dérivé de 1,5AnFru, tel qu'une de ses formes tautomères et ou un de ses hydrates, ou le produit de déshydratation du 1,5AnFru, ou d'une de ses formes tautomères et d'un de ses hydrates, ou leurs dérivés.

7. Utilisation suivant l'une quelconque des revendications précédentes, dans laquelle ledit éther cyclique est le 1,5AnFru.

8. Utilisation suivant l'une quelconque des revendications précédentes, dans laquelle ledit médicament est destiné au traitement du diabète.

9. Composition destinée à être utilisée en médecine, ladite composition comprenant :
i) un éther cyclique comme ingrédient actif, ledit éther cyclique étant l'éther cyclique tel que défini dans l'une quelconque des revendications 1 à 8 ; et facultativement
ii) un support, diluant ou excipient pharmaceutiquement acceptable ;
dans laquelle ledit éther cyclique est capable d'avoir un effet sur la tolérance au glucose.

10. Composition suivant la revendication 9, ladite composition étant destinée à moduler GLP-1 et/ou à traiter un trouble de tolérance au glucose, tel que le diabète.

11. Procédé pour la préparation d'une composition telle que définie dans l'une quelconque des revendications 9 à 10, ledit procédé comprenant les étapes consistant :
i) à fournir un éther cyclique tel que défini dans l'une quelconque des revendications 1 à 8 ; et
ii) à mélanger ledit éther cyclique avec un support, diluant ou excipient pharmaceutiquement acceptable.

12. Procédé suivant la revendication 11, dans lequel ladite composition est destinée à être utilisée pour agir sur un trouble de tolérance au glucose et/ou pour moduler GLP-1.

13. Utilisation de 1,5AnFru dans la production d'un médicament destiné à agir sur un trouble de tolérance au glucose.
